Europäisches Patentamt

European Patent Office

Office européen des brevets

(11) Numéro de publication: **0 318 356**
**A1**

# DEMANDE DE BREVET EUROPEEN

(21) Numéro de dépôt: **88402865.5**

(22) Date de dépôt: **15.11.88**

(51) Int. Cl.4: **A 61 F 5/02**
**A 61 B 17/58, C 21 D 7/10**

(30) Priorité: **23.11.87 FR 8716209**

(43) Date de publication de la demande:
**31.05.89 Bulletin 89/22**

(84) Etats contractants désignés: **DE ES GB IT**

(71) Demandeur: **SOCIETE DE FABRICATION DE MATERIEL ORTHOPEDIQUE**
**60-62 rue Rothschild**
**F-62600 Berk/Mer (FR)**

(72) Inventeur: **Cotrel, Yves**
**110 Rue Félix Faure**
**F-75015 Paris (FR)**

(74) Mandataire: **Martin, Jean-Paul et al**
**c/0 CABINET LAVOIX 2, Place d'Estienne d'Orves**
**F-75441 Paris Cedex 09 (FR)**

(54) Dispositif pour l'étaiement des vertèbres du rachis.

(57) Dispositif pour l'étaiement du rachis, comportant au moins une tige (4) ainsi que plusieurs organes d'ancrage (7) pouvant prendre appui sur des vertèbres du rachis et pourvus de moyens de blocage sur ladite tige (4); ces moyens de blocage comprennent au moins une vis pointeau (1) écrouie, et choisie en un matériau ne créant pas d'effet galvanique avec la tige (4) ni avec les organes d'ancrage (7). L'écrouissage de la vis pointeau (1) lui confère une dureté supérieure à celle de la tige (4) et de l'organe d'ancrage (7), ce qui améliore la tenue mécanique du dispositif.

FIG.3

EP 0 318 356 A1

Bundesdruckerei Berlin

Description

## Dispositif pour l'étaiement des vertèbres du rachis

La présente invention a pour objet un dispositif pour l'étaiement du rachis, mis en place par intervention opératoire, ainsi qu'une vis faisant partie de ce dispositif.

Ce dipositif peut être utilisé pour simplement étayer un rachis qui en a besoin (fracture accidentelle par exemple) ou bien encore pour redresser et étayer un rachis objet de déviations (scolioses, cyphoses par exemple).

On connait divers systèmes d'instrumentation et d'étaiement du rachis, notamment COTREL-DUBOUSSET décrit par la demande de brevet européen 128058, qui comporte des tiges sur lesquelles plusieurs organes d'ancrage peuvent prendre appui sur des vertèbres du rachis, et qui sont pourvus de moyens de blocage sur ladite tige. Ces moyens comprennent des vis de pression à têtes hexagonales qui exercent un effort sur la surface de la tige.

Pour que l'ensemble de ce dispositif puisse être implanté sur le rachis sans risque de corrosion galvanique, il est obligatoire d'utiliser le même matériau pour tous les éléments : tige, organes d'ancrage et vis de blocage. De plus, ce matériau ne doit pas être écroui afin de ne pas modifier localement ses caractères électrochimiques et par conséquent d'empêcher toute apparition d'une différence de potentiel locale dans le métal, à l'endroit de l'écrouissage avec le reste du métal inaltéré. De ce fait, le métal non écroui a une dureté relativement faible, de sorte que les vis de blocage subissent des déformations indésirables après leur serrage, le dispositif ne donnant donc pas entière satisfaction au point de vue de sa tenue mécanique.

De plus, les tetes hexagonales des vis uti lisées soulèvent d'autres difficultés : si après leur mise en place, le chirurgien désire conserver une possibilité ultérieure de démontage du dispositif, les têtes doivent être laissées en place et font saillie des organes d'ancrage. Elles augmentent donc l'encombrement de ceux-ci, et de plus sont susceptibles de créer une gêne pour le patient. Si pour éviter ces inconvénients, le chirurgien sépare les têtes hexagonales du reste des vis après mise en place celles-ci, en exerçant un couple de torsion suffisant, les vis ne peuvent plus être ultérieurement retirées, et par conséquent le dispositif ne peut plus être démonté, même si un tel démontage s'avère souhaitable.

L'invention a pour but de remédier à ces divers inconvénients.

Suivant l'invention, les moyens de blocage des organes d'ancrage comprennent une vis pointeau écrouie et choisie en un matériau ne créant pas d'effet galvanique avec la tige, ni avec les organes d'ancrage.

La vis pointeau peut être réalisée en un tel matériau convenablement choisi, par exemple l'acier inoxydable correspondant à la norme américaine 316L (norme AFNOR Z 2 CND 17.13) avec une teneur en molybdène de 2,5 à 3%, élaboré sous vide, par écrouissage. Cette vis présente une dureté très supérieure à celle des vis utilisées jusqu'à présent, sans que pour autant apparaisse un effet de pile entre une telle vis écrouie et les autres éléments du dispositif d'étaiement, réalisés dans le même matériau mais non écrouis.

La pointe écrouie de la vis pointeau a donc une dureté nettement supérieure à celle de la tige dans laquelle elle s'enfonce sans déformation, ce qui assure une excellente tenue mécanique au dispositif.

En effet, on peut vérifier par des essais que la résistance de la vis pointeau au glissement sur la tige et sur les crochets d'ancrage est notablement supérieure à celle des vis utilisées jusqu'à présent.

De préférence, la vis pointeau est écrouie par déformation à froid.

D'autres particularités et avantages de l'invention apparaitront au cours de la description qui va suivre, faite en référence aux dessins annexés qui en illustrent une forme de réalisation à titre d'exemple non limitatif :

- la Figure 1 est une vue en élévation avec arrachement partiel d'une forme de réalisation de la vis pointeau selon l'invention;

- la Figure 2 est une vue suivant la flèche K de la Figure 1 de l'extrémité de la vis pointeau;

- la Figure 3 est une vue mi-coupe partielle, mi-élévation d'une partie d'un dispositif pour l'étaiement du rachis, comprenant une tige et plusieurs organes d'ancrage similaires, dont un seul est représenté et muni d'une vis pointeau de blocage selon l'invention;

- la Figure 4 est une vue mi-coupe, mi-élévation d'un appareil d'essais de résistance au glissement longitudinal de vis pointeau équipant un dispositif d'étaiement du rachis selon l'invention;

- la Figure 5 est un diagramme illustrant un essai de résistance au glissement d'une vis pointeau selon l'invention et de vis antérieurement utilisées;

- la Figure 6 est un diagramme similaire à celui de la Figure 5, mais relatif à un dispositif équipé de deux vis pointeaux de blocage.

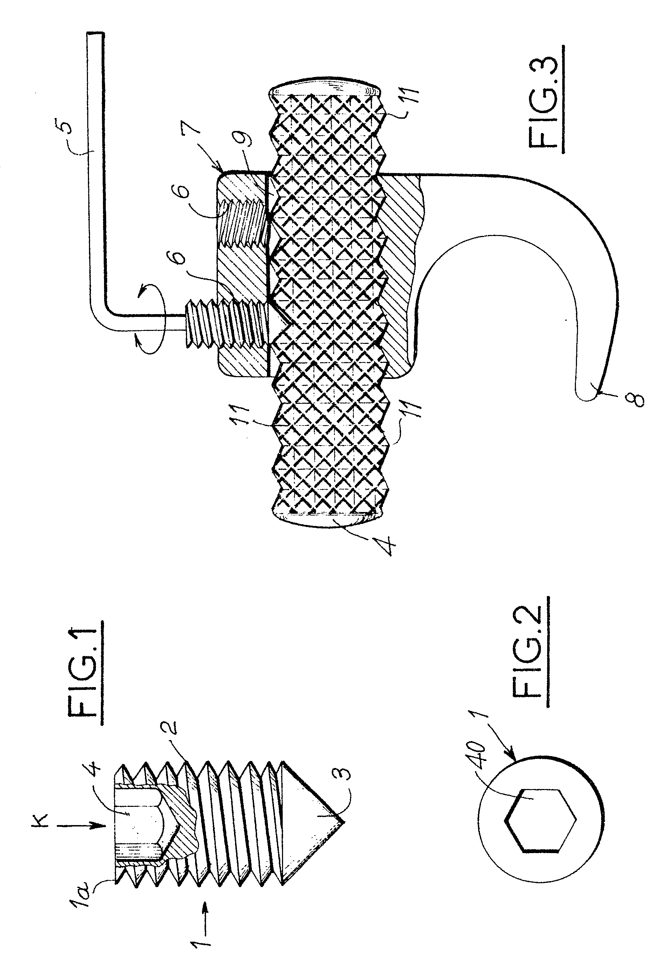

La vis représentée aux Figures 1 et 2 est une vis pointeau destinée à servir de moyen de blocage d'un organe d'ancrage d'un dispositif d'étaiement du rachis, tel que celui décrit dans la demande de brevet européen précitée.

La vis 1 comporte une tige filetée 2 munie d'une pointe terminale 3, cette vis 1 étant écrouie et réalisée en un matériau ne créant pas d'effet galvanique, ni avec la tige ni avec l'organe d'ancrage avec lequel cette vis est en contact, ceux-ci étant formés dans le même matériau que la vis 1.

Il est de plus prévu, dans la partie postérieure 1a de la vis 1, opposée à sa pointe 3, un logement 4 profilé pour pouvoir recevoir un outil 5 (Figure 3) de serrage ou de desserrage dans un trou taraudé 6 d'un organe 7 comportant un crochet 8 d'ancrage dans une vertèbre non représentée.

Dans l'exemple décrit, le logement 4 a un profil à six pans et peut naturellement être profilé de manière différente. L'organe d'ancrage 7 est percé d'un canal 9 traversé par la tige-support 4, dont la surface peut être lisse ou présenter des aspérités 11 entre lesquelles peut s'enfoncer la pointe 3 de la vis pointeau 1, par manoeuvre de l'outil 5. L'organe d'ancrage 7 est percé de deux trous taraudés 6 destinés à recevoir chacun une vis 1 ou, en variante, d'un seul trou 6.

La vis 1, l'organe d'ancrage 7 et la tige 4 sont réalisés dans le même matériau approprié, tel que celui-mentionné ci-dessus, choisi de telle sorte que, la tige 4 et l'organe 7 n'étant pas écrouis, et la vis pointeau 1 étant par contre écrouie, par exemple par déformation à froid, aucun effet galvanique (ou effet de pile) n'apparaisse par suite de l'écrouissage de la vis 1 entre celle-ci d'une part, et la surface de la tige 4 ainsi que l'organe 7 d'autre part.

La partie postérieure de la vis 1, dans laquelle est ménagé le logement 4, est dimensionnée de manière à affleurer sensiblement la surface de l'organe d'ancrage 7 après vissage complet dans celui-ci et enfoncement de sa pointe 3 dans la tige 4. Cet enfoncement est possible sans déformation de la vis 1, grâce au fait que sa dureté est supérieure à celle de la tige 4 et à celle de l'organe d'ancrage 7. Ceci améliore considérablement la tenue mécanique d'un dispositif d'étaiement dont les organes d'ancrage 7 sont équipés des vis pointeaux 1, par rapport au dispositif décrit dans la demande de brevet européen précitée.

Par ailleurs, l'absence de saillie de la partie postérieure 1a de la vis 1 par rapport à la surface de l'organe d'ancrage 7 élimine les problèmes d'encombrement et de gêne pour le patient créés par les vis antérieures, tout en permettant un démontage ultérieur du dispositif par le chirurgien en cas de besoin.

Les aspérités 11 formant le moletage de la surface de la tige 4 améliorent la fixation de cette dernière aux organes d'ancrage 7 par pénétration dans la paroi du canal 9, pour la bloquer en rotation et en translation. Elles peuvent être, comme connu en soi, constituées notamment par des pointes de diamant. Cependant, la fabrication de ce type de tige à pointes de diamant exige un tel degré de déformation du métal qu'il est pratiquement impossible de garantir une fiabilité totale contre toute présence de criques, de microfissurations et d'incrustations d'éléments étrangers. C'est pourquoi une particularité de l'invention prévoit que les aspérités 11 peuvent être réalisées par déformation à froid ou par enlèvement de matière. Ainsi, la déformation à froid peut être exécutée par exemple par moletage, tandis que l'enlèvement de matière peut être réalisé par usinage ou encore par meulage.

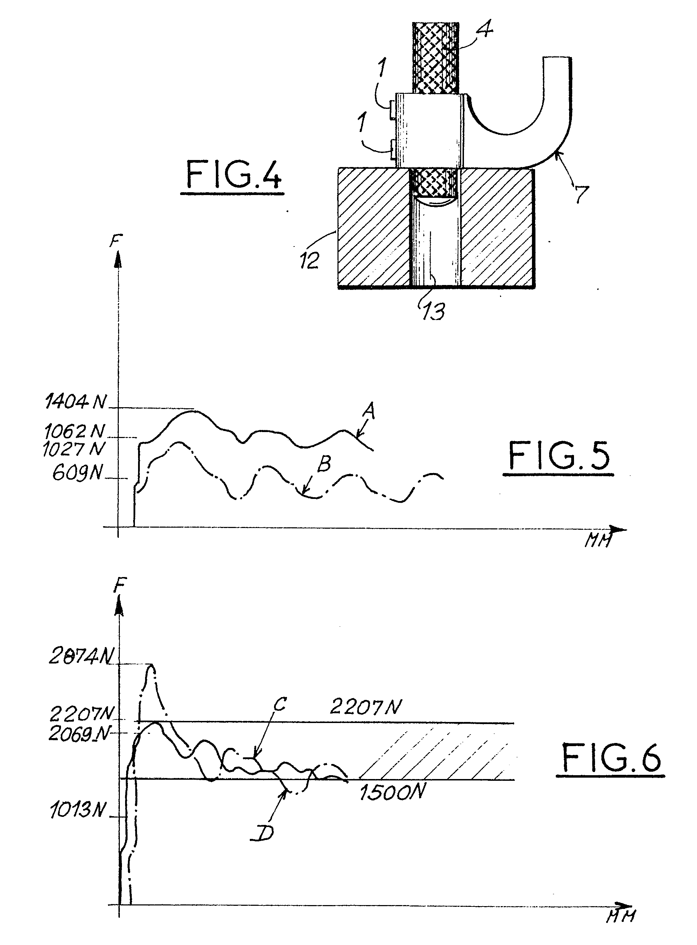

La Figure 4 montre schématiquement un système d'essai de résistance au glissement de deux vis pointeaux 1 selon l'invention, par rapport à la tige 4. Ce système comporte un bloc support 12 percé d'un puits central 13 dans lequel pénètre la tige 4, l'organe d'ancrage 7 prenant appui sur la surface du bloc 12. Dans cet exemple, l'organe 7 est muni de deux vis pointeaux 1, l'essai pouvant également être effectué avec une seule vis 1. On exerce sur la tige 4 une force axiale F vers le puits 13, cette force tendant à faire glisser la tige 4 sur les pointes 3 enfoncées entre les aspérités 11.

Un premier essai étant effectué avec une seule vis pointeau 1, mise en place dans 1 organe 7 avec un couple de serrage de 0,82mN, couple maximum avant la limite de déformation du profil 40 à six pans, on constate (Figure 5, courbe A) qu'aucun glissement significatif ne se produit entre la tige 4 et la vis 1 jusqu'à une force F = 1404 N. La courbe B de la Figure 5 montre, à titre comparatif avec une vis classique à tête hexagonale non écrouie, que le glissement apparaît, en fonction des caractéristiques de cette vis, à partir de forces nettement inférieures à 1404 Newtons.

Un essai de glissement longitudinal avec deux vis pointeaux 1 équipant l'organe d'ancrage 7, serré comme la précédente vis par un couple de 0,82 mN, permet de constater (Figure 6) qu'un glissement significatif n'apparaît que pour une force F = 2207 N.

On voit sur la zone hachurée que le glissement (en mm) augmente, tandis que la force F diminue après le pic de 2207 N, Pour se stabiliser vers 1500 N (courbe C). La courbe D présente un pic de 2874 N et correspond à la variation du glissement en fonction de la force appliquée à la tige 4 traversant un organe d'ancrage 7 muni de deux vis hexagonales classiques.

Le fait que le logement 4 de la vis pointeau 1 selon l'invention permette un démontage ultérieur du du dispositif d'étaiement sans pour autant créer une partie proéminente gênante sur la surface de l'organe d'ancrage, constitue un avantage important vis-à-vis de la technique antérieure. Ainsi par exemple chez l'enfant, cela permet de retendre le montage après un laps de temps durant lequel une croissance progressive de l'enfant entraîne une moins bonne tension du montage, et ce, sans que pendant cette période, l'enfant ne soit gêné par des tetes hexagonales proéminentes de vis.

Enfin, tout matériau approprié, autre que l'acier 316L écroui mentionné ci-dessus, peut être utilisé dans le cadre de l'invention. Il convient également de noter que la dureté de la tige 4 est supérieure à celle des organes d'ancrage 7. La dureté de la tige 4 est par ailleurs inférieure à celle de la vis pointeau 1, comme déjà mentionné.

**Revendications**

1 - Dispositif Pour l'étaiement du rachis, comportant au moins une tige (4) et plusieurs organes d'ancrage (7) pouvant prendre appui sur des vertèbres du rachis et pourvus de moyens de blocage sur ladite tige, caractérisé en ce que les moyens de blocage comprennent au moins une vis pointeau (1) écrouie, choisie en un materiau ne créant pas d'effet galvanique avec la tige (4) ni avec les organes d'ancrage (7).

2 - Dispositif selon la revendication 1, caractérisé en ce que la vis pointeau (1) est réalisée dans le même matériau que les organes d'ancrage (7) et la tige (4), par exemple de l'acier inoxydable écroui 316 L.

3 - Dispositif selon l'une des revendications 1 et 2, caractérisé en ce que la dureté de la vis pointeau (1) est supérieure à celle de la tige (4) et des organes d'ancrage (7) et est obtenue par déformation à froid.

4 - Dispositif selon l'une des revendications 1 à 3, caractérisé en ce que la vis (1) présente une pointe (3) destinée à pénétrer dans la tige (4), et dans sa partie postérieure (1a) opposée à la pointe, un logement (4) profilé pour pouvoir recevoir un outil (5) de serrage ou de desserrage.

5 - Dispositif selon la revendication 4, caractérisé en ce que la partie postérieure (1a) de la vis (1) est dimensionnée de manière à affleurer sensiblement la surface de l'organe d'ancrage (7) après vissage dans celui-ci et enfoncement de sa pointe (3) dans la tige (4).

6 - Dispositif selon l'une des revendications 1 à 5, caractérisé en ce que la surface de la tige (4) comporte des aspérités (11), améliorant sa fixation aux organes d'ancrage (7) pour les bloquer en rotation et en translation, et réalisées par déformation à froid ou par enlèvement de matière.

7 - Dispositif selon l'une des revendications 1 à 6, caractérisé en ce que la dureté de la tige (4) est supérieure à celle des organes d'ancrage (7) et inférieure à celle de la vis pointeau (1).

8 - Vis (1) destinée à un dispositif d'étaiement du rachis selon l'une des revendications 1 à 7, caractérisée en ce qu'elle présente une pointe (3) de pénétration dans la tige (4) et est réalisée en un matériau écroui ne créant pas d'effet galvanique avec la tige (4) et l'organe d'ancrage (7) associés.

FIG.1

FIG.2

FIG.3

FIG.4

FIG.5

FIG.6

Office européen
des brevets

RAPPORT DE RECHERCHE EUROPEENNE

Numero de la demande

EP 88 40 2865

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y,D | EP-A-0 128 058 (SOFAMOR)<br>* Page 8, ligne 4 - page 9, ligne 32; résumé; figures 4-12 * | 1,3,6 | A 61 F 5/02<br>A 61 B 17/58<br>C 21 D 7/10 |
| Y | DE-B-1 017 195 (TROSS)<br>* Colonne 3, lignes 3-45 * | 1,3,6 | |
| A | | 8 | |
| A | EP-A-0 240 376 (HARDY)<br>* Colonne 5, ligne 44 - colonne 6, ligne 11; figure 4 * | 1,4,5,8 | |
| A | FR-A-2 348 275 (MANNESMAN)<br>* Page 1, lignes 1-17; page 3, lignes 16-30 * | 1,3,8 | |
| A | FR-A-2 369 825 (V.N.I.I.M.I.)<br>* Page 11, lignes 4-10; page 12, lignes 4-11; figures 3,6,8 * | 1,5 | |
| A | EP-A-0 028 985 (UNION CARBIDE)<br>* Page 1, ligne 1 - page 3, ligne 32; résumé * | 2,3 | DOMAINES TECHNIQUES RECHERCHES (Int. Cl.4) |
| A | MECANIQUE, no. 289, janvier 1984, pages 35-39; F. ROUSSEAU: "Aciers inoxydables à hautes caractéristiques" | | A 61 F<br>A 61 B<br>B 21 K<br>C 21 D |
| A | US-A-4 229 875 (CRISPELL) | | |
| A | WO-A-8 701 026 (OLERUD) | | |

Le présent rapport a été établi pour toutes les revendications

| Lieu de la recherche | Date d'achèvement de la recherche | Examinateur |
|---|---|---|
| LA HAYE | 21-02-1989 | KLEIN C. |